# EUROPEAN PATENT APPLICATION

(11) **EP 4 383 483 A1**
(43) Date of publication of application: **12.06.2024**
(21) Application number: 22211959.6
(22) Date of filing: 07.12.2022
(51) Int. Cl.: H01S 3/00, H01S 3/23, H01S 3/16, A61F 9/008, H01S 3/10, H01S 3/11

(54) **A LASER**

(71) Applicant: NKT Photonics A/S, 3460 Birkerød (DK)
(72) Inventor: SEIFERT, Albert, 8105 Regensdorf (CH)
(74) Representative: Zacco Denmark A/S

(57) **Abstract**

A laser configured to selectively operate in two different operational modes is disclosed. In the first operational mode the laser is configured to output femtosecond laser pulses and in the second operational mode the laser is configured to output nanosecond laser pulses.

## Description

### TECHNICAL FIELD

The invention relates to the technical field of lasers configured to operate in two different modes.

### BACKGROUND

In some industrial applications, for example in material science, material processing, as well as in some medical applications, such as ophthalmology or genetics, several lasers are used to carry out one or more tasks on a sample piece. The provision of several lasers may be costly and may require a complicated setup.

It is an object of the present invention to provide a simpler solution.

### SUMMARY OF THE INVENTION

The object is satisfied by a laser in accordance with claim 1. Preferred embodiments of the invention are described in the dependent claims.

In a first aspect, a laser configured to selectively operate in two different operational modes, a first operational mode and a second operational mode, is disclosed. In the first operational mode the laser is configured to output femtosecond laser pulses. In the second operational mode the laser is configured to output nanosecond laser pulses.

The femtosecond pulses may have a duration in the range between 50 fs up to 1000 fs, such as in the range between 300 fs up to 450 fs. The femtosecond pulses may have an average power between 1 Watt and 1 Kilowatt, such as between 2-100 Watt, such as 5 Watt. The femtosecond pulses may have peak powers as high as 20 Petawatt, such as 1-10 GW, such as 100 kW. The femtosecond pulses may have a repetition rate as high as 1 MHz, such as in the range between 1 kHz to 1 MHz.

The nanosecond pulses may have a duration in the range between 5 ns up to 500 ns, such as in the range between 30 ns up to 100 ns. The nanosecond pulses may have an average power up to 1 Kilowatt, such as between 2-100 Watt, such as 5 Watt. The nanosecond pulses may have peak powers as high as 90 MW. Such as 88 MW, such as 100 kW. The nanosecond pulses may have a repetition rate as high as 10 MHz, such as in the range between 1 kHz to 5 MHz, such as 1 MHz.

The laser may therefore be configured to selectively provide wide pulses in the ns range and comparatively narrower pulses, at least 1000 times narrower than the pulses in the ns range.

The laser may be configured to deliver high average power up to 1 kW, such as high as between 2-100 Watt, such as 5 W regardless of the operational mode. The laser may be configured to deliver pulses with a repetition rate up to 10 MHz, such as in the range between 1 kHz to 5 MHz, such as around 1 MHz, regardless of the operational mode.

In some embodiments, the laser may comprise a plurality of components for generating and/or processing the femtosecond laser pulses and the nanosecond laser pulses. At least a portion of the plurality of components is used in both operational modes.

In some embodiments, the laser may comprise an oscillator. In some embodiments, the laser may comprise a pulse stretcher. In some embodiments, the laser may comprise an amplifier. The amplifier may comprise a cavity. In some embodiments the laser may comprise a pulse compressor. In some embodiments the laser may comprise a controller.

In some embodiments the laser may comprise a modulator. The modulator may be configured to operate as a shutter.

In some embodiments, in the first operational mode, the amplifier may be configured to operate in an amplification mode and provide amplified pulses.

In the first operational mode, the pulse compressor may be configured to compress the amplified pulses temporally down to thereby produce femtosecond pulses. The compressor may be a dispersive compressor. The compressor may be a grating pair or a chirped volume Bragg grating. The compressor may temporally compress the pulses to a duration similar to the pulse duration of the initial seed pulses. In the second operational mode, the compressor may not significantly influence the nanosecond pulses. For instance, the compressor may have a temporal dispersion between 100-150 ps, such as 120-130 ps. When such dispersion is added to ns pulses, e.g. 5-500 ns pulse width, the compression is insignificant. Even if the compressor has a larger dispersion, e.g. about 1-2 ns such dispersion will still be insignificant for the nanosecond pulses. The compressor may be disabled and/or bypassed in the second operational mode.

The modulator, e.g. a shutter, may be configured to modulate the energy of the amplified pulses. The modulator may be configured to control the pulse emission. The modulator may be an acousto-optical modulator. The modulator may be an electro-optical modulator. The modulator may be selected depending on a laser application, i.e. on requirements for laser pulse energy and/or repetition rate. The modulator may be configured to decrease the repetition rate of the pulses.

In some embodiments, in the second operational mode, the amplifier may comprise a cavity configured to generate nanosecond pulses. Thereby, in the second operational mode, the amplifier may be operated in a mode that does not require any external optical signal input into the amplifier. Rather, the amplifier may, by utilizing its resonator cavity, i.e. multiple passes of light pulses through the cavity, generate nanosecond pulses having high peak power, such as between 10 kW to 100 kW, depending on the repetition rate. In the second operational mode, the amplifier may deliver up to 100 W of average output power. In the second operational mode, the amplifier may generate 5 ns to 500 nanosecond pulses, such as 5-500 ns, such as 10-300 ns, such as 150 ns. In the second operational mode the pulses may be provided with high power by the amplifier, and may then be only manipulated in terms of repetition rate and wavelength. Pulse power and/or pulse energy can be modulated by the modulator, such as by a process shutter.

In some embodiments, the amplifier may be a regenerative amplifier. The regenerative amplifier may be operated in different modes, depending on desired output pulses, their duration, peak power, and repetition rate. For the first operational mode, the regenerative amplifier may be fed by seed pulses which are then amplified, while in the second operational mode, the regenerative amplifier may be operating in a laser oscillator mode. The regenerative amplifier typically achieves amplification through multiple passes of light pulses through a laser gain medium (e.g. a solid-state medium). The gain medium may be placed in an optical resonator, together with an optical switch, which is usually realized with an electro-optic modulator and a polarizer. Using a regenerative amplifier is beneficial as this amplifier can provide high amplification of short pulses and can be operated in different modes.

In some embodiments, when operating the laser in the second operational mode, the nanosecond pulses may be generated by the amplifier's cavity using cavity-dumping principle. The basic idea with the cavity-dumping principle is that the optical losses of the amplifier's cavity are kept as low as possible for some time. This gives time for an intense light pulse build-up in the cavity. This pulse can then be extracted within about one cavity round-trip time using a cavity dumper, such as an acousto-optical modulator or an electro-optical modulator, such as a Pockels cell.

Alternatively, when operating the laser in the second operational mode, the nanosecond pulses may be generated by the amplifier's cavity using a Q-switching principle. In this case, intracavity losses are modulated and thus the Q factor of the amplifier's cavity, to thereby obtain nanosecond pulses. Furthermore, it is also possible to combine the cavity-dumping technique with Q-switching to thereby generate the nanosecond pulses by the amplifier's cavity.

In some embodiments, the modulator may be arranged after the amplifier. The modulator may be an acousto-optical or an electro-optical modulator. The modulator may be configured to control the pulse emission as well as to modulate the pulse energy. The modulator can control both the femtosecond pulses and the nanosecond pulses. The modulator may be arranged either before or after the compressor, irrespective of the mode the amplifier is operating in. The modulator may be controlled to the adapt peak power and repetition rate of the pulses.

In some embodiments, the laser may further include an oscillator, in particular a short pulse oscillator, and a pulse stretcher. In the first operational mode, the oscillator may be configured to generate femtosecond pulses. The oscillator may be a fiber oscillator or a free-space oscillator. The oscillator may incorporate an Ytterbium doped KYW active laser crystal. The oscillator may also incorporate other Ytterbium doped crystals, such as Yb:YAG, Yb:KGW, Yb:CALGO, Yb:Lu203. The oscillator may generate pulses with a high repetition rate, such as between 20-100 MHz, such as 80 MHz. The oscillator may be arranged before the amplifier. The oscillator may provide seed pulses. The oscillator may provide pulses to the amplifier. The oscillator may be configured to provide femtosecond pulses. In the second operational mode, the oscillator may be disabled or blocked. The fs pulses generated by the oscillator may be amplified before being output from the laser.

The pulse stretcher may be configured to generate temporally stretched pulses from the femtosecond pulses. The pulses may be stretched to e.g. 100 ps, or even to 1-2 ns. In particular, the pulse stretcher may be configured to generate temporally stretched pulses in the picosecond to nanosecond range. The pulse stretcher may further generate chirped pulses. The pulse stretcher may comprise a dispersive element. The pulse stretcher may be a fiber-Bragg grating, a chirped-volume Bragg grating, a long fiber (e.g. 10 m long or more), a diffraction grating-based stretcher, such as a Martinez stretcher. The Martinez stretcher may include two lenses placed between two gratings. By adjusting the distances between the gratings and the lenses the dispersion of the stretcher can be tuned.

The stretcher may be arranged between the oscillator and the amplifier, such that, in the first operational mode, it stretches the pulses generated by the oscillator and provides the stretched pulses to the amplifier. The stretcher may not influence the repetition rate of the pulses. In the second operational mode, the stretcher may be inactive. The pulse stretcher is beneficial as it may reduce the peak power of the pulses such that detrimental effects in the amplifier, receiving the stretched pulses, are avoided.

The stretched pulses are typically amplified and then compressed. The pulse compressor may be an element with dispersion opposite to the dispersion of the stretcher, to thereby remove the chirp and temporally compress the pulses to a duration similar to the duration of the pulses input to the stretcher.

In some embodiments, in the first operational mode, the amplifier may be configured to receive the temporally stretched pulses and to generate the amplified pulses from the temporally stretched pulses. The long pulse duration of the stretched pulses reduces the peak power to a level where detrimental effects in the amplifier gain medium are avoided. Preferably, the amplified pulses have a reduced repetition rate compared to the repetition rate of the temporally stretched pulses. The repetition rate may be reduced to the kHz range. The amplifier, comprising e.g. a Pockels cell defining a round trip number of the pulses in the amplifier, may control the repetition rate of the pulses. The highest pulse energies are achieved at lower repetition rates. Typically, the repetition rate of amplified pulses may be between 50 kHz and 1 MHz, such as between 100-500 kHz.

In some embodiments, the laser further comprises an output switch. The switch may be configured to direct pulses either directly to an output of the laser or to redirect the pulses for further optical processing. The output switch may be arranged after the modulator or after the pulse compressor.

In some embodiments the laser may comprise a harmonics generation component. The harmonics generation component may comprise one or two crystals, e.g. LBO and/or BBO crystals, converting the pulses to the second, third, fourth harmonic. In the second operational mode, the output switch may be configured to direct the nanosecond pulses to the harmonics generation component. The harmonics generation component may be configured to generate harmonics from the nanosecond pulses and to output the generated harmonics. The harmonics generation component may be configured to change the central wavelength of the pulses. The pulses with changed, typically lower, wavelength compared to the initial seed pulses may then be sent to the laser output. The laser output in this case may be an output of the harmonics generation component. The harmonics generation component may have a plurality of outputs for outputting pulses with different central wavelength. It may be beneficial to arrange the harmonics generation component in the laser to thereby add further functionalities to the laser, such as generation of different wavelengths. In some examples, the harmonics generation component may convert pulses in the infra-red (IR) range to ultra-violet (UV) range. In some examples, the harmonics generation component may convert IR pulses to pulses in green spectral range. To convert IR pulses to UV pulses, two crystals may be required. The harmonic generation component may change pulse duration. Typically, the harmonic generation component may change average power of the pulses as the conversion efficiency is typically lower than 100%.

In some embodiments, the harmonics generation component is bypassed in the first operational mode or in the second operational mode. The harmonics generation component may be optimized differently for ns pulses compared to configuration for fs pulses.

In some embodiments, the output switch may be synchronised with the oscillator. The output switch may be coupled with the oscillator to synchronize the output of the femtosecond pulses at the output switch with the generation of the femtosecond pulses at the oscillator. The output switch may be electrically coupled to the oscillator. The synchronization may be established via an electronic controller.

In some embodiments, the modulator, e.g. a shutter, may be synchronized with the oscillator and/or with the output switch. The modulator may be coupled with the output switch and the oscillator for controlling the emission of the femtosecond pulses and/or for modulating the pulse energy of the femtosecond pulses. The modulator may be synchronised with the oscillator/switch by the electronic controller. The modulator may be electrically coupled with the output switch and the oscillator through the electronic controller. Synchronization between the oscillator, modulator, and the output switch may be beneficial for overall performance of the laser and may ensure that good-quality pulses with high peak power are generated. In some embodiments, the harmonic generation component may be synchronized with the amplifier, and/or the oscillator.

In some embodiments, the laser comprises a controller, in particular an electronic controller, configured to switch the operational mode of the laser between the first operational mode and the second operational mode. The electronic controller may disable components which are not used in pulse generation in one of the modes. The electronic controller may ensure synchronous operation of various components forming part of the laser. The electronic controller may be operated by a user to thereby change the operational mode of the laser. It is beneficial to have an electronic controller operated by the user to ensure easy operation of the laser. The electronic controller may be operated by an external controller allowing the laser to be integrated in a system with the system determining the operation mode of the laser.

In some embodiments, the controller is coupled, in particular electrically coupled, with the amplifier to switch the operation of the amplifier between an amplification mode and a cavity dumping or Q-switching operational mode. Having an amplifier with dual operational mode allows for a laser with two different operational modes. By switching the operational mode of the amplifier, the operational mode of the laser is switched. The controller may be the same electronic controller configured for controlling other components of the laser such that all components are controlled simultaneously to thereby ensure the change of the operational mode of the laser.

The electronic controller may be a mode selector operationally connected with the laser components to define an operational mode for the laser. The controller may be connected to a computer which may control the operational mode of the laser as required by application in which the laser is used. The computer may further control pulse duration, repetition rate, central wavelength, etc.

In some embodiments, the femtosecond pulses are generated by chirped-pulse-amplification in the laser. It is advantageous to use chirped pulse amplification for generation of the femtosecond pulses as this technique allows for amplifying pulses to very high optical intensities while avoiding excessive nonlinear pulse distortions or optical damage. The amplified femtosecond pulses may be generated from seed femtosecond pulses which, before passing through the amplifier, are chirped and temporally stretched to a much longer duration in the pulse stretcher. The pulses are then sent to the amplifier. The long pulse duration reduces the peak power to a level where undesired nonlinear effects in the amplifier are avoided. After amplification, an element, e.g. a compressor, which removes the chirp and temporally compresses the pulses is used. The chirped-pulse-amplification can provide that pulses have a duration similar to the pulse duration of the seed pulses while their peak power is significantly increased.

In some embodiments, the femtosecond pulses have a central wavelength at about 1030 nm. The femtosecond pulses may have a central wavelength at about 1050 nm. The central wavelength of the femtosecond pulses may be defined by the oscillator generating seed pulses. The central wavelength of the femtosecond pulses may depend on the active laser medium used in the oscillator, generating the seed pulses. The central wavelength of the femtosecond pulses may be influenced by the amplifier.

In some embodiments, the nanosecond pulses have a central wavelength in the green or in the UV spectral range after passing through the harmonic generation component. The central wavelength of the nanosecond pulses may be defined by conversion in the harmonic generation component. In some examples, the central wavelength of the nanosecond pulses may be 1030 nm, or 1050 nm. Having a laser configured to provide pulses with a number of different wavelengths is beneficial as it allows various uses of the laser in different fields.

In some embodiments, the central wavelength of the laser pulses can be adapted by the harmonic generation component. Both the nanosecond pulses and the femtosecond pulses may be processed by the harmonic generation component. The harmonic generation component may be selected depending on the application of the laser. Alternatively, the laser may comprise a nanosecond harmonic generation component suitable for conversion of nanosecond pulses and a femtosecond harmonic generation component suitable for conversion of the femtosecond pulses. Depending on the operation mode of the laser, one of the components will be bypassed while the other one will be used for conversion.

In a second aspect, a medical system comprising the laser in accordance with the first aspect is disclosed. The medical system may further comprise means for guiding the laser beam from the laser to a patient. The means for guiding the laser beam may include various optical components. The system may further comprise a controller for controlling both the laser and the means for guiding the laser beam.

In some embodiments, the medical system for use in ophthalmologic procedures is disclosed. The medical system may therefore be an ophthalmologic system.

In some embodiments, the medical system for use in surgical procedures is disclosed. The medical system may be an ophthalmologic surgical system.

The above aspects, accompanying claims, and/or examples disclosed herein above and later below may be suitably combined with each other as would be apparent to anyone of ordinary skill in the art.

Additional features and advantages are disclosed in the following description, claims, and drawings, and in part will be readily apparent therefrom to those skilled in the art or recognized by practicing the disclosure as described herein.

### BRIEF DESCRIPTION OF THE DRAWINGS

Preferred embodiments of the present invention will now be described by way of example only and with reference to the accompanying drawings in which:
Fig. 1 shows an exemplary embodiment of a laser according to the present disclosure.
Fig. 2 shows an exemplary embodiment of a laser according to the present disclosure.
Fig. 3 shows an exemplary embodiment of a laser operating in the first operational mode.
Fig. 4 shows an exemplary embodiment of a laser operating in the second operational mode.
Fig. 5 shows yet another exemplary embodiment of a laser according to the present disclosure.
Fig. 6 shows yet another exemplary embodiment of a laser according to the present disclosure.
Fig. 7 shows yet another exemplary embodiment of a laser according to the present disclosure.
Fig. 8 shows yet another exemplary embodiment of a laser according to the present disclosure.

### DETAILED DESCRIPTION

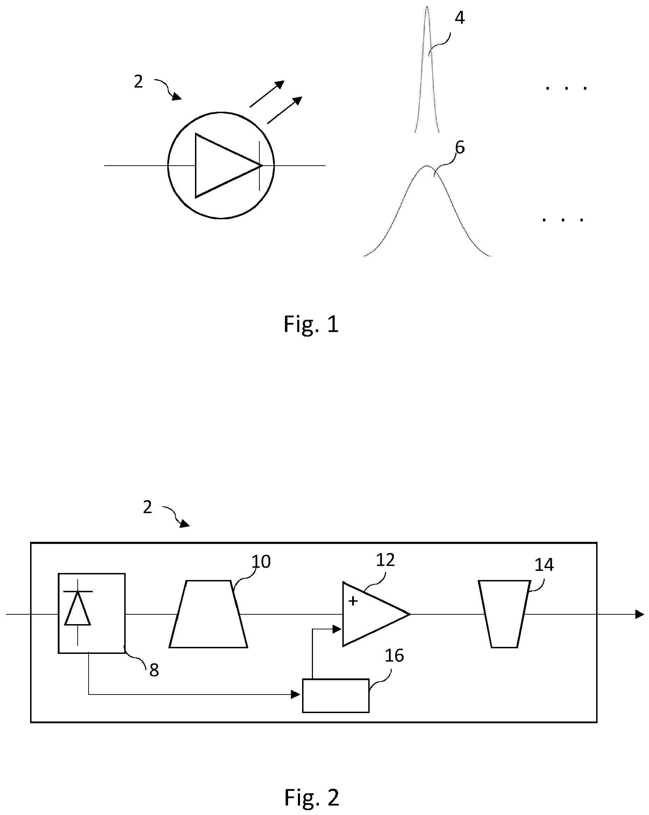

Fig. 1 shows an exemplary embodiment of a laser 2 according to the present disclosure. The laser is configured to selectively operate in two different operational modes. In the first operational mode the laser is configured to output femtosecond laser pulses 4 and in the second operational mode the laser is configured to output nanosecond laser pulses 6.

Fig. 2 shows a more detailed exemplary embodiment of a laser 2 according to the present disclosure. The laser 2 comprises a plurality of components for generating and/or processing the femtosecond laser pulses and the nanosecond laser pulses and at least some of the plurality of components are used in both operational modes. In the embodiment shown in Fig. 2, the laser 2 comprises an oscillator 8, a pulse stretcher 10, an amplifier 12, a pulse compressor 14, and a controller 16.

The oscillator 8 may generate seed pulses. The seed pulses may have a central wavelength at around 1030 nm.

The controller 16 may be configured to switch the operational mode of the laser 2 between the first operational mode and the second operational mode. The electronic controller 16 may disable components which are not used in pulse generation in one of the modes. The electronic controller 16 may ensure synchronous operation of various components forming part of the laser. The electronic controller may be operated by a user to thereby change the operational mode of the laser 2. It is beneficial to have an electronic controller operated by the user to ensure easy operation of the laser.

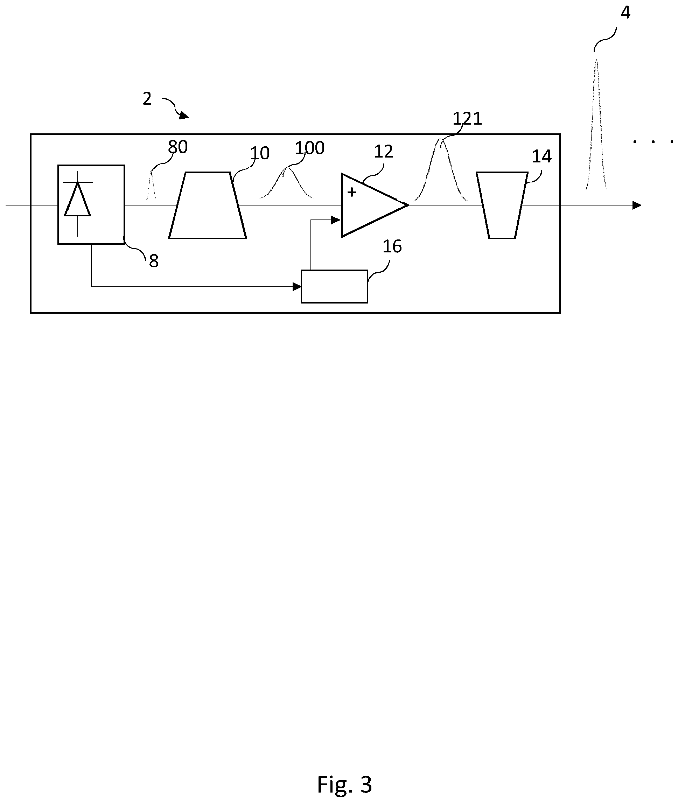

Fig. 3 shows an exemplary embodiment of a laser operating in the first operational mode. In the first operational mode, the oscillator 8 is configured to generate short pulses 80, such as femtosecond pulses, or picosecond pulses below 10 ps, such as 1-2 ps pulses. The repetition rate of the pulses may be high, such as 80 MHz or higher. The pulse stretcher 10 is configured to generate temporally stretched pulses 100 from the short pulses 80. The temporally stretched pulses 100 may be in the picosecond or nanosecond range. The temporally stretched pulses 100 are input into the amplifier 12. In the first operational mode, the amplifier 12 is configured to amplify the received pulses and output amplified pulses 121. The controller 16 may control the amplifier 12 to operate in a standard, amplification, regime. The amplifier 12 may be a regenerative amplifier. The amplifier 12 may reduce the repetition rate of the input pulses. Finally, the amplified pulses 121 are compressed in the compressor 14. The pulse compressor 14 is configured to compress the amplified pulses 121 temporally down to the femtosecond range, thereby generating femtosecond pulses 4.

The femtosecond pulses 4 may be generated by chirped-pulse-amplification in the laser 2. It is advantageous to use chirped pulse amplification for generation of the femtosecond pulses 4 as this technique allows for amplifying pulses to very high optical intensities while avoiding excessive nonlinear pulse distortions or optical damage. The amplified femtosecond pulses 4 may be generated from seed femtosecond pulses 80 which, before passing through the amplifier 12, are chirped and temporally stretched, in the pulse stretcher 10, to a much longer duration, e.g. ps or ns. The stretched pulses 100 are then sent to the amplifier 12. The long pulse duration reduces the peak power to a level where undesired nonlinear effects in the amplifier 12 are avoided. After amplification, the compressor 14 removes the chirp and temporally compresses the pulses. The fs pulses 4 have a duration similar to the pulse duration of the seed pulses 80 while their peak power is significantly increased, as schematically illustrated in Fig. 3.

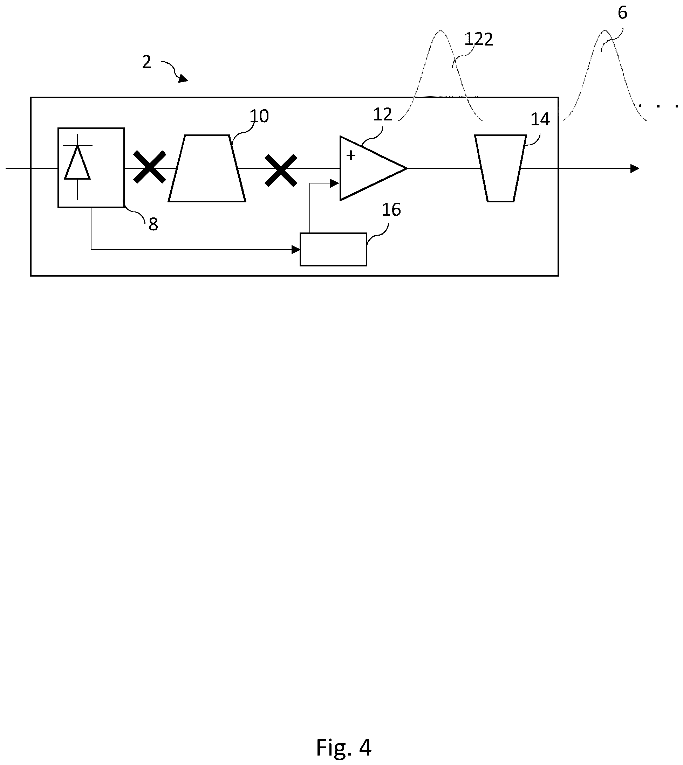

Fig. 4 shows an exemplary embodiment of a laser 2 operating in the second operational mode. In the second operational mode, the oscillator 8 is blocked or disabled. This can be controlled via the controller 16. Naturally, the pulse stretcher 10 will, having no input, also be inactive. In the second operational mode, the amplifier 12 may comprise a cavity configured to generate nanosecond pulses 122. Thereby, in the second operational mode, the amplifier 12 may be controlled by the controller 16, to operate in a mode that does not require any external optical input (a laser oscillator mode). Rather, the amplifier 12 will, by utilizing its resonator cavity, i.e. multiple passes of light pulses through the cavity, generate nanosecond pulses 122 having high peak power (e.g. 10 kW to 100 kW). In the second operational mode, the nanosecond pulses 122 may be generated by the amplifier's cavity using cavity-dumping principle. Alternatively, when operating the laser 2 in the second operational mode, the nanosecond pulses 122 may be generated by the amplifier's cavity using a Q-switching principle. Furthermore, it is also possible to combine cavity-dumping technique with Q-switching to thereby generate the nanosecond pulses by the amplifier's cavity. In the second operational mode, the pulse compressor 14 has a negligible effect on relatively long ns pulses 122. The nanosecond pulses 6 are as such output from the laser 2.

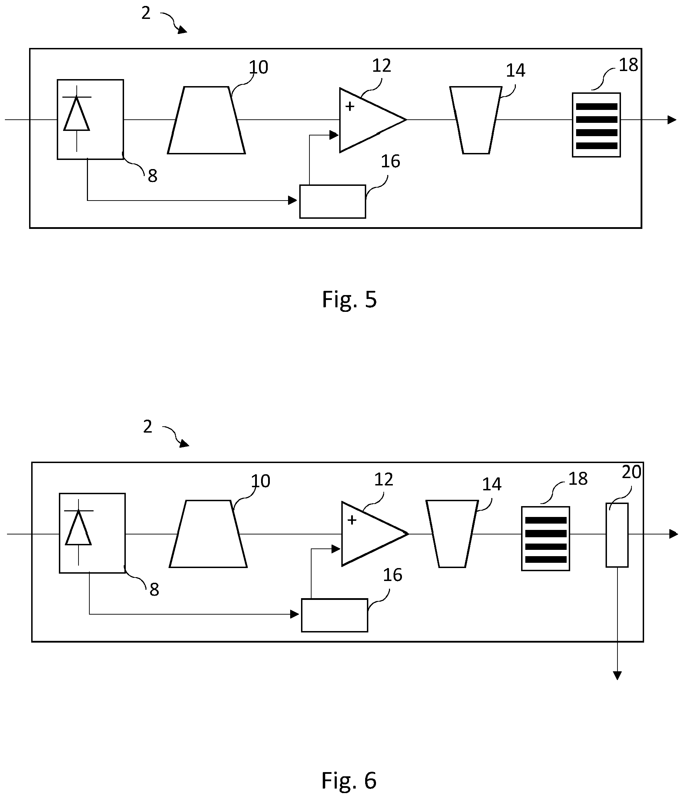

Fig. 5 shows yet another exemplary embodiment of a laser 2 according to the present disclosure. The laser 2 comprises all the components as elaborated in connection to Figs. 2-4 and additionally comprises a modulator 18. The modulator 18 may be configured to operate as a shutter. The modulator may be arranged after the pulse compressor 14 (as shown in Fig. 5). Alternatively, the modulator 18 may be coupled in the system after the amplifier 12. The placement of the modulator is independent of the mode the amplifier 12 is operating in. The modulator may be an acousto-optical or an electro-optical modulator. The modulator may be configured to control the pulse emission as well as to modulate the pulse energy. The modulator can control both the femtosecond pulses and the nanosecond pulses. The modulator may be controlled to adapt peak power and repetition rate of the pulses.

Fig. 6 shows yet another exemplary embodiment of a laser 2 according to the present disclosure. The laser 2 comprises all the components as elaborated in connection to Figs. 2-5 and additionally comprises an output switch 20. The output switch may be coupled to the modulator. In some embodiments, the modulator 18 may be omitted and the switch may therefore be coupled to the compressor 14. The output switch may have two separate outputs, one for outputting femtosecond pulses, and another one for outputting nanosecond pulses.

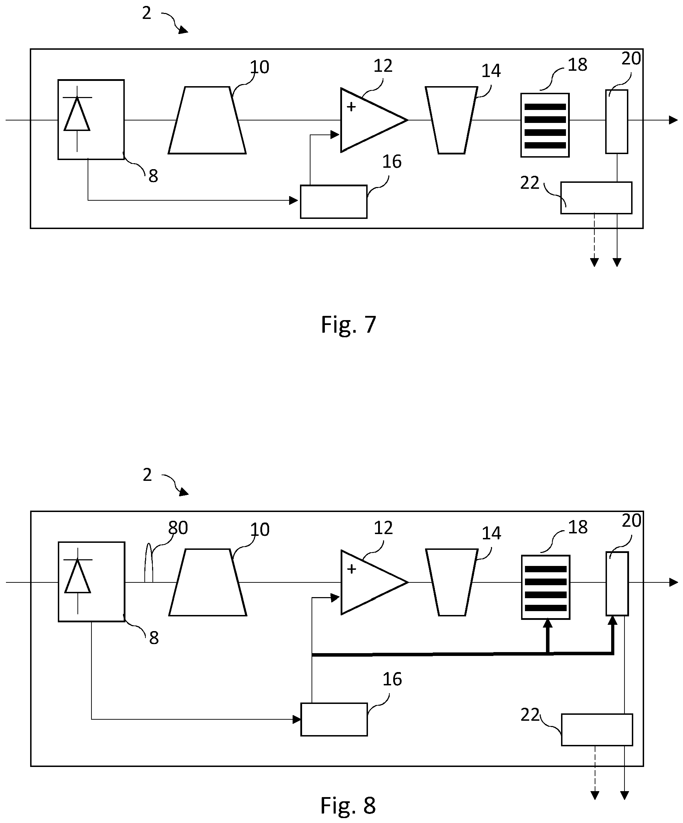

Fig. 7 shows yet another exemplary embodiment of a laser 2 according to the present disclosure. The laser 2 comprises all the components as elaborated in connection to Figs. 2-6 and additionally comprises a harmonics generation component 22. The harmonics generation component 22 may be coupled to one of the outputs of the output switch 20, such as to the nanosecond output. In some embodiments, the modulator 18 and the switch 20 may be omitted and the harmonics generation component 22 may therefore be directly coupled to the compressor 14. The harmonics generation component may have two separate outputs, one for outputting pulses in the UV range, and another one for outputting pulses in the green spectral range.

Fig. 8 shows yet another exemplary embodiment of a laser 2 according to the present disclosure. In this embodiment, the amplifier 12 is coupled with the oscillator 8, via the controller 16, in order to ensure clean picking in and out of the amplifier. The output switch 20 is electrically coupled with the amplifier 12, to synchronize the output pulses at the output switch 20 with the generation of the seed pulses 80 at the oscillator 8. Further, the modulator 18 may be electrically coupled with the output switch 20 and the amplifier 12 for controlling the emission of the output pulses and/or for modulating the pulse energy of the output pulses.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

Relative terms such as "below" or "above" or "upper" or "lower" may be used herein to describe a relationship of one value to another value. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the inventive concepts being set forth in the following claims.

### Reference signs

- 2: laser
- 4: femtosecond pulses
- 6: nanosecond pulses
- 8: oscillator
- 80: short pulses generated by the oscillator
- 10: pulse stretcher
- 100: stretched pulses
- 12: amplifier
- 121: amplified pulses
- 122: amplified ns pulses
- 14: pulse compressor
- 16: controller
- 18: modulator
- 20: output switch
- 22: harmonics generation component

## Claims

1. A laser configured to selectively operate in two different operational modes, wherein in the first operational mode the laser is configured to output femtosecond laser pulses and in the second operational mode the laser is configured to output nanosecond laser pulses.

2. The laser according to claim 1, wherein the laser comprises a plurality of components for generating and/or processing the femtosecond laser pulses and the nanosecond laser pulses and wherein at least a portion of the plurality of components is used in both operational modes.

3. The laser according to claim 1 or 2, wherein the laser comprises an oscillator, a pulse stretcher, an amplifier comprising a cavity, and a pulse compressor.

4. The laser according to claim 3, wherein, in the first operational mode, the amplifier is configured to operate in an amplification mode and provide amplified pulses, the amplifier optionally being a regenerative amplifier,
the pulse compressor being configured to compress the amplified pulses temporally down to produce femtosecond pulses, and
wherein the laser further comprises a modulator, the modulator being configured to modulate the energy of the amplified pulses.

5. The laser according to claim 3 or 4, wherein, in the second operational mode, the cavity of the amplifier is configured to generate nanosecond pulses, and wherein the nanosecond pulses are generated by the amplifier's cavity using cavity-dumping principle or Q-switching principle.

6. The laser according to claim 4 or 5, wherein the modulator is an acousto-optical or an electro-optical modulator arranged after the amplifier.

7. The laser according to any one of claims 3 to 6, wherein the oscillator is a short pulse oscillator, wherein, in the first operational mode, the oscillator is configured to generate femtosecond pulses, and
wherein the pulse stretcher is configured to generate temporally stretched pulses from the femtosecond pulsed, in particular temporally stretched pulses in the picosecond to nanosecond range.

8. The laser according to claim 7, wherein, in the first operational mode, the amplifier is configured to receive the temporally stretched pulses and to generate the amplified pulses from the temporally stretched pulses, wherein, preferably, the amplified pulses have a reduced repetition rate with regard to the repetition rate of the temporally stretched pulses.

9. The laser according to any of the preceding claims, wherein the laser further comprises a harmonics generation component and an output switch, and wherein in the second operational mode, the output switch is configured to direct the nanosecond pulses to the harmonics generation component, which is configured to generate harmonics from the nanosecond pulses and to output the generated harmonics.

10. The laser according to claim 9, wherein the harmonics generation component is bypassed in the first operational mode or in the second operational mode, and wherein a central wavelength of the laser pulses is adapted by the harmonic generation component.

11. The laser according to claim 9 or 10, wherein the output switch is coupled, in particular electrically coupled, with the oscillator to synchronize the output of the femtosecond pulses at the output switch with the generation of the femtosecond pulses at the oscillator, wherein, optionally, the modulator is coupled, in particular electrically coupled, with the output switch and the oscillator for controlling the emission of the femtosecond pulses and/or for modulating the pulse energy of the femtosecond pulses.

12. The laser according to any one of the preceding claims, wherein the laser comprises a controller, in particular an electronic controller, configured to switch the operational mode of the laser between the first operational mode and the second operational mode.

13. The laser according to claims 13, wherein the controller is coupled, in particular electrically coupled, with the amplifier to switch the operation of the amplifier between an amplification mode and a cavity dumping or Q-switching operational mode.

14. The laser according to any of the preceding claims, wherein the femtosecond pulses are generated by chirped-pulse-amplification in the laser, and/or wherein the femtosecond pulses have a central wavelength at about 1030 nm, and wherein the nanosecond pulses have a central wavelength in the green or in the UV spectral range.

15. A medical system comprising the laser according to any of the preceding claims.
